Europäisches Patentamt

European Patent Office

Office européen des brevets

(11) Publication number : **0 607 017 A1**

# (12) EUROPEAN PATENT APPLICATION

(21) Application number : **94300181.8**

(22) Date of filing : **11.01.94**

(51) Int. Cl.⁵ : **A61F 2/30**

(30) Priority : **11.01.93 JP 19285/93**

(43) Date of publication of application :
**20.07.94 Bulletin 94/29**

(84) Designated Contracting States :
**DE FR GB**

(71) Applicant : **TDK CORPORATION**
**13-1, Nihonbashi 1-chome**
**Chuo-ku Tokyo (JP)**

(72) Inventor : **Nonami, Tohro, c/o TDK Corporation**
**13-1, Nihonbashi 1-chome,**
**Chuo-ku**
**Tokyo (JP)**
Inventor : **Satoh, Naoyoshi, c/o TDK**
**Corporation**
**13-1, Nihonbashi 1-chome,**
**Chuo-ku**
**Tokyo (JP)**
Inventor : **Yamazaki, Junichi, c/o TDK**
**Corporation**
**13-1, Nihonbashi 1-chome,**
**Chuo-ku**
**Tokyo (JP)**

(74) Representative : **Adams, William Gordon et al**
**RAWORTH, MOSS & COOK**
**36 Sydenham Road**
**Croydon Surrey CR0 2EF (GB)**

(54) **Composite biotic implant and method of making a composite biotic implant.**

(57) A composite biotic implant having improved bio-affinity and high durability in the living body is provided. Ceramic particles are embedded in a metal substrate to form an embedment layer wherein the particles are received in recesses in the substrate. In a cross section of the embedment layer, at least 5% of the recesses satisfy A < B wherein A is the distance between opposed ends of the outside edge of the recess, and B is the maximum diameter of the ceramic particle. Alternatively or additionally, at least 5% of the recesses satisfy that at least one end of the outside edge of the recess has an included angle θ of less than 90°.

FIG. 1a

(a)

FIG. 1b

(b)

Jouve, 18, rue Saint-Denis, 75001 PARIS

## FIELD OF THE INVENTION

This invention relates to a composite biotic implant suitable as artificial dental roots, dental crowns, bones, and joints as well as a method for preparing the same.

## BACKGROUND OF THE INVENTION

Biotic implants are advantageously utilized as bio-replacements or complements to be implanted in living tissues or cavities, for example, artificial dental roots and crowns, artificial bones, artificial joints, bone fillers, artificial blood vessels, etc.

As biotic implants meeting both mechanical strength and bio-affinity, there were proposed several composite implants comprising a metal substrate coated on the surface with bio-affinity ceramics, for example, implants having hydroxyapatite plasma sprayed on metal as disclosed in Japanese Patent Publication (JP-B) No. 39533/1983, metal implants which are oxidized on the surface and coated with calcium phosphate as disclosed in JP-B 6537/1990, 14060/1990, 14061/1990 and 18102/1990, and implants having a graded structure as disclosed in Japanese Patent Application Kokai (JP-A) No. 147455/1988.

These implants, however, have the risk that since the bond between a metal substrate and a ceramic material is established only by chemical reaction at their interface, the ceramic material can chip away or peel off due to weak bond strength. It is a serious problem that once chipping or peeling occurs at small areas, the ceramic layer can eventually peel off over a large extent.

For implants as artificial dental roots, it is important to control the surface nature and roughness of the implants for facilitating their integration to living bones and growth of neoblastic bones. The conventional methods, however, are difficult to achieve desired surface nature while maintaining strength.

The applicant previously proposed a composite biotic implant comprising a metal substrate having ceramic particles embedded in a surface thereof and preferably covered with a layer of ceramic material in Japanese Patent Application No. 45676/1991 (USSN 07/839,391 and EP 92 905106.8 filed February 20, 1992). In this proposal, ceramic particles are embedded in a metal substrate by virtue of metal plasticity to induce physical and chemical bonds, thereby providing an implant possessing both strength and bio-compatibility.

This proposal, however, has a risk that ceramic particles can fall off in the living body since only the embedment depth of ceramic particles is controlled and thus physical anchoring of ceramic particles to the metal substrate is weak. Some ceramic materials are likely to be leached in the living body. Particles of such ceramic material are more likely to fall off from the metal substrate because of leaching in the vicinity of their interface with the substrate.

Even when ceramic particles are embedded to a sufficient depth, there can be left gaps between ceramic particles and the outside edge of recesses in the metal substrate where the particles are received. When a ceramic coating is formed over the substrate, it cannot fill in such gaps and the gaps are left as voids, which in turn, causes the ceramic coating to separate from the substrate more frequently.

## SUMMARY OF THE INVENTION

An object of the present invention is to provide a composite biotic implant having improved bio-affinity and high durability in the living body.

The present invention provides a composite biotic implant comprising a metal substrate having a surface layer where ceramic particles are embedded and received in recesses. According to one form of the present invention, in a cross section of the embedment layer, at least 5%, preferably at least 10% of the recesses satisfy A < B wherein A is the distance between opposed ends of the outside edge of the recess in which a ceramic particle is received, and B is the maximum diameter of the ceramic particle as measured perpendicular to a normal to the metal substrate surface.

According to another form of the present invention, in a cross section of the embedment layer, at least 5%, preferably at least 10% of the recesses satisfy that at least one of opposed ends of the outside edge of the recess has an included angle of less than 90°.

In one preferred embodiment, the metal substrate is made of a metal exhibiting a ductility of at least 50% at a temperature corresponding to 70% or less of its melting point.

In another preferred embodiment, the implant further includes a ceramic coating layer disposed on the embedment layer. The ceramic coating layer has a non-calcium phosphate system composition containing silicon oxide, calcium oxide, and magnesium oxide wherein diopside crystals occupy at least 50% by volume of the entire crystalline matter. The surface layer of the metal substrate includes a diffusion stratum containing oxygen and silicon.

Preferably, the composite biotic implant is prepared using a hot press assembly including a mold defining a cavity and a punch. Ceramic particles are applied to the surface of a metal substrate. The metal substrate is fully inserted into the mold cavity. The punch is forced against the substrate and into the mold cavity to uniaxially pressurize the metal substrate, thereby embedding the ceramic particles in the metal substrate. When the metal substrate has been fully inserted into the mold cavity, the ceramic particles on the surface are spaced a distance of up to 0.5 mm, preferably 0.05 to 0.4 mm from the mold cavity surface.

The present invention uses a highly plastic metal having high strength and ductility as the substrate in a surface of which ceramic particles are embedded by a metal plastic working process. The metal substrate and the ceramic particles are joined through mechanical engagement, achieving a very high bond strength which has never been achieved in the prior art. Differently stated, the ceramic particles do not leave the metal substrate unless the particles themselves are crushed. Since discrete ceramic particles are independently joined to the metal substrate, only some ceramic particles separately fall out even if chipping occurs and it never happens that the coating layer peels off in "plane" cleavage as in the prior art.

Since ceramic particles are embedded in the metal substrate surface, the implant surface is embossed and roughened at the same time as junction, ensuring anchoring effects of promoting initial fixation and neoblastic bone growth.

Moreover, in the embodiment in which an implant having ceramic particles embedded therein is surface coated with a biotic ceramic material, the spaces between ceramic particles are filled with the ceramic material, thus preventing the metal components from being leached out, resulting in further increased bio-compatibility, bio-affinity and bio-activity. In this embodiment, if the material of the ceramic particles and the material of the ceramic coating layer are of substantially the same type, the coating layer is integrally joined to the ceramic particles in mechanical engagement with the metal substrate, resulting in a significantly high bond strength.

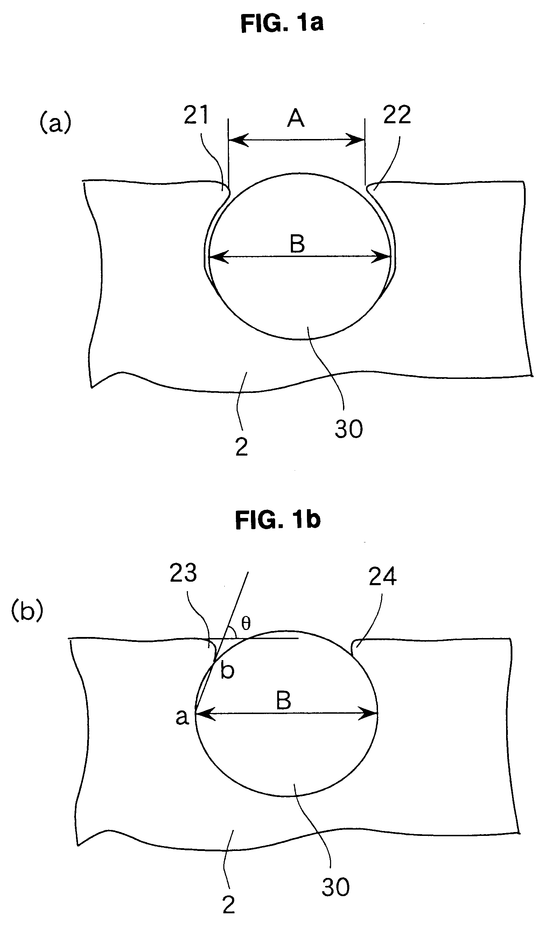

According to the present invention, in a cross section of the ceramic particle embedment layer as shown in FIG. 1, at least 5% of the recesses satisfy A < B wherein A is the distance between opposed ends of the outside edge of the recess in which a ceramic particle is received, and B is the maximum diameter of the ceramic particle as measured perpendicular to the metal substrate normal line. This prevents escape or disengagement of ceramic particles in normal conditions and even when ceramic particles are slightly dissolved away into smaller size particles. Since the gaps between the ceramic particles and the metal substrate into which the particles are embedded are reduced, a ceramic coating layer can be easily formed over the entire surface of the substrate. This ensures that the ceramic coating layer does not separate away from the substrate.

## BRIEF DESCRIPTION OF THE DRAWINGS

FIGS. 1a and 1b are schematic cross-sectional views of ceramic particle embedment layer.

FIG. 2 is an exploded perspective view illustrating a process for preparing a composite biotic implant according to the present invention.

FIG. 3a is a cross-sectional elevation of a hot press assembly used in the manufacture of a composite biotic implant according to the present invention.

FIG. 3b is a cross-sectional elevation of a conventional hot press assembly.

FIG. 4 is a ternary diagram of $SiO_2$-CaO-MgO system for explaining the composition of preferred ceramic material.

FIG. 5 is a SEM photomicrograph in a cross section of a ceramic particle embedment surface layer according to the present invention.

FIG. 6 is a SEM photomicrograph in a cross section of a ceramic particle embedment surface layer in the prior art.

FIG. 7 is a schematic cross-sectional view of a part of a substrate including a ceramic particle embedment layer and covered with a ceramic coating layer.

## DETAILED DESCRIPTION OF THE INVENTION

The composite biotic implant of the present invention includes a metal substrate having a surface layer where ceramic particles are embedded. This surface layer is also referred to as an embedment layer.

## [Metal substrate]

The metal material used as the metal substrate is not particularly limited insofar as it is not harmful to the living body. Preferably, it has a strength in excess of a certain level, for example, at least 500 MPa, and exhibits "high plasticity," that is, significant plasticity at or below the temperature at which strength is significantly reduced, for example, the temperature corresponding to 70% of the melting point. Preferred among others are highly plastic metal materials having a ductility of at least 50% at a temperature corresponding to 70% or less of its melting point and a pressure of 50 MPa, more preferably superplastic metal materials having a ductility of at least 200% under these conditions because a joint is more easily established between the metal and ceramic particles when the ceramic particles are embedded in a surface layer of the metal substrate.

Preferred examples of the superplastic metal material include titanium (Ti) and titanium base alloys containing up to 20% by weight in total of at least one element selected from the group consisting of Al, V, Fe, Mo, Cr, Zr, Pd, N, Si, N and O, for example, Ti-6Al-4V (expressed in % by weight), Ti-Al-Sn, Ti-Pd, Ti-Mo, Ti-Zr, Ti-Fe, Ti-Al-V-Mo-Fe, Ti-Fe-N-O, Ti-Cr-Si, and Ti-Pd-Cr; Al base alloys such as Zn-22Al, Zn-21.5Al-0.01Mg alloy (SPZ); stainless steel; and Ni base alloys. With respect to bio-compatibility, however, pure titanium (ductility about 200%) and titanium base alloys are preferred. The Ti and Ti alloys generally have a coefficient of thermal expansion $\alpha_1$ of approximately $8\text{-}10 \times 10^{-6}/°C$.

It will be understood that the metal substrate can have a flat surface since it is deformed by the subsequent embedding process although it may have a previously roughened surface. The surface roughness may range from the measurement lower limit to 300 $\mu$m as measured according to JIS B-0601.

[Embedment layer]

Referring to FIGS. 1a and 1b, the embedment layer is schematically illustrated in cross section. In FIG. 1a, a metal substrate 2 is formed with a recess 20 in which a ceramic particle 30 is received or embedded. Only one recess is shown for convenience sake although a plurality of recesses are formed in the substrate 2. The recess 20 is open at the substrate surface and thus has a generally circular outside (upper in the figure) edge which appears at opposed ends 21 and 22 in the cross section. The opposed ends 21 and 22 are spaced a distance A. The ceramic particle 30 has a maximum diameter B as measured perpendicular to a normal line N to the metal substrate surface. (Note that this normal line is aligned with a radial line from the center of the substrate when it is a cylindrical or conical one.) In one form of the present invention, at least 5%, preferably at least 10% of the entire recesses satisfy A < B, preferably $1.01A \leqq B$. If the proportion of those recesses with A < B among the entire recesses is less than 5%, no benefits of the invention are achieved. This proportion is preferably determined by cutting the embedment layer typically in a radial plane, randomly picking up more than 100 recesses appearing in the cross section, and measuring the relevant sizes of the recesses and particles.

In the other form of the invention, at least 5%, preferably at least 10% of the entire recesses in which ceramic particles 30 are received or embedded satisfy that at least one of the opposed ends 23 and 24 of the outside edge of the recess 20 has an included angle $\theta$ of less than 90°, preferably up to 85°. The included angle $\theta$ is defined as the angle between a plane perpendicular to a normal line N to the metal substrate surface and line a-b, provided that $\underline{a}$ is the intersection between a normal line N to the metal substrate surface and the maximum diameter B of the ceramic particle 30 as measured perpendicular to the normal line N and $\underline{b}$ is the point at which the ceramic particle 30 is free from the metal substrate 2 as shown in FIG. 1b. If the proportion of those recesses with $\theta$ < 90° among the entire recesses is less than 5%, no benefits of the invention are achieved. This proportion is preferably determined by cutting the embedment layer typically in a radial plane, randomly picking up more than 100 recesses appearing in the cross section, and measuring the angle associated with the recesses and particles.

Of course, it is advantageous that recesses and ceramic particles satisfy both the requirements prescribed in accordance with FIGS. 1a and 1b, that is, A < B and $\theta$ < 90°.

The embedment depth of ceramic particles or percent embedment is preferably 55 to 100%, more preferably 60 to 70% of the particle diameter. At a too low percent embedment, less recesses satisfy one or both of the above-mentioned requirements. At a too higher percent embedment, the implant anchoring effect is reduced. The percent embedment may be calculated by observing a cross section of an implant under a microscope, randomly picking up 100 embedded particles, and determining the ratio of the embedment distance to the particle length in a direction normal to the substrate.

Further, the percent of the area of an implant covered with ceramic particles relative to the area of the implant in contact with a living hard tissue (biotic contact area) optionally through a ceramic coating layer, that is, coverage by ceramic particles is preferably at least 20%, more preferably at least 40%, most preferably at least 50%.

It is to be noted that the process of embedding ceramic particles does not substantially deform the ceramic particles, but the metal substrate. The amount of deformation in a substrate surface layer corresponds to the aforementioned percent embedment. Since ceramic particles are not substantially deformed by the embedding process, an embedment layer is generally formed by a single layered arrangement of embedded particles.

The embedment layer of ceramic particles should preferably have a surface roughness of 1 to 2,000 $\mu$m, especially 10 to 300 $\mu$m (Ra defined by JIS B-0601). A too smooth embedment layer allows for slippage on the surface and provides little anchoring effect whereas a too rough embedment layer gives a reduced area of contact with the adjoining bone retarding the rate of integration.

[Embedding]

The implant of the present invention is manufactured by embedding ceramic particles in a surface layer of the metal substrate. The technique used herein may be a "metal plastic working process" of applying heat and pressure to ceramic particles in contact with a metal substrate, thereby achieving embedment and joint by taking advantage of the plastic deformation of the metal. Inter alia, plastic or superplastic working is preferred.

The metal plastic working process may be performed by means of a conventional hot press (HP), hot isostatic press (HIP), warm isostatic press (WIP) or the like. HP is preferably used for implants of simple shape because of ease of operation. HIP and WIP are used for implants of complex shape.

The pressing temperature is generally in the range of from 200 to 1,200°C and below the melting point of the metal material as the substrate. Pressing is preferably carried out in a non-oxidizing atmosphere, typically vacuum in order to prevent oxidation of the metal substrate. If the metal substrate is little affected by oxidation, the atmosphere need not be limited to a non-oxidizing one. The pressure is preferably 1 to 500 MPa, more preferably 5 to 300 MPa, most preferably 50 to 200 MPa. The working time is generally from 1 to 600 minutes. The amount of deformation is generally at least 0.1, preferably about 1 to 3 as expressed in true strain of the overall substrate although it varies with the size of particles to be buried.

Plastic working is performed on a metal substrate of a predetermined shape which has been coated with grease, for example, over a necessary area and has ceramic particles adhered to the substrate.

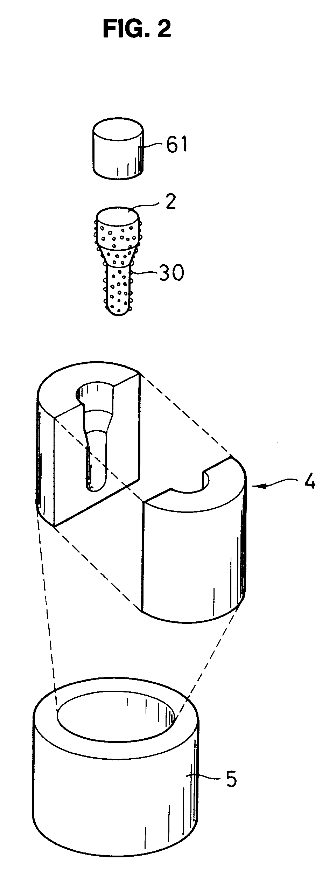

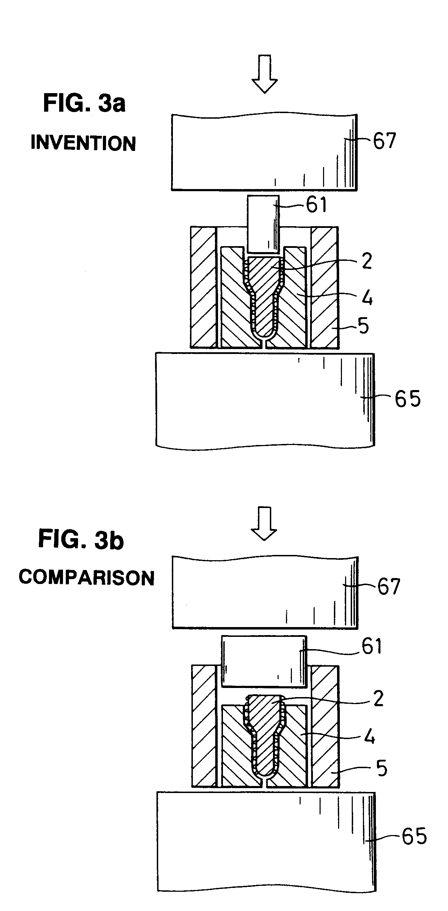

FIGS. 2 and 3a illustrate a plastic working process. First, high vacuum grease is applied to the surface of a metal substrate 2 and ceramic particles 30 are adhered to the entire side and bottom surfaces of substrate 2. Hot pressing is effected uniaxially in vacuum by means of a hot press for radially extending the metal substrate for providing plastic working. The hot press assembly used is shown in FIGS. 2 and 3a as including a split mold 4 received in a cylindrical sleeve 5 on a support 65, an upper punch 61 disposed above the split mold 4, and press means 67 for pressing the punch 61 against the support 65.

In order that the recesses in which ceramic particles are embedded be configured as defined herein, the hot press assembly shown in FIG. 3a is preferably used. The metal substrate 2 is completely inserted into the cavity of the split mold 4. That is, the entire substrate 2 is within the confine of the mold cavity. The punch 61 is forced against the substrate and into the mold cavity whereby the metal substrate is uniaxially pressurized. This assembly allows plastic deformation of the metal substrate under pressure to be effectively utilized for embedding ceramic particles and thus facilitates formation of recesses to the desired configuration.

In the hot press assembly, when the metal substrate is fully inserted into the mold cavity, the ceramic particles on the substrate surface are preferably spaced a distance of up to 0.5 mm, more preferably up to 0.2 mm from the mold cavity surface. Most often the spacing is from 0.05 to 0.4 mm. If the ceramic particles are spaced more than 0.5 mm from mold cavity surface, it becomes difficult to form ceramic particle-receiving recesses to the desired configuration.

Preferably the metal substrate has a higher coefficient of thermal expansion than that of the mold. Where a titanium substrate (8x10$^{-6}$/°C) is used, for example, molds of $Si_3N_4$ (3x10$^{-6}$/°C), SiC (5x10$^{-6}$/°C) and $Al_2O_3$ are preferred.

[Ceramic particles]

The ceramic particles used herein generally have a mean particle size of from 1 to 2,000 $\mu$m, preferably from 30 to 300 $\mu$m. Within this range, plastic embedment is likely to occur. Too large particle sizes would render plastic embedment difficult and leave a larger space between particles, and larger particles result in an implant of larger dimensions. Too small particle sizes would render embedment operation difficult. The use of ceramic particles within the above-defined particle size range ensures the manufacture of implants having a desired surface roughness as will be described later.

Preferably, the ceramic particles have a particle size distribution as uniform as possible. Ceramic particles with a uniform particle size are embedded to a uniform depth (percent embedment), which leads to the uniformity of bond strength. It is to be noted that if relatively large particles are used, small particles may be used in combination to form a mixture having two or more particle size distributions. In this case, small particles intervene between large particles when embedded, imparting uniformity to the embedment layer in a plane direction.

As to geometry, the ceramic particles should preferably have an average shape factor of up to 1.2. As the shape factor increases, the shape becomes more irregular so that ceramic particles can be locally distributed on the substrate leaving sparse areas. For this reason, a shape factor approximate to unity is preferred. It is to be noted that the shape factor is the maximum diameter divided by the minimum diameter of a particle and the average shape factor is calculated by randomly picking up about 100 particles.

Further ceramic particles of spherical shape are preferred because such particles are slidingly displaced during pressing with the likelihood to form a embedment layer which is even or uniform in a plane direction. Also after an implant is implanted in a living tissue, the ceramic layer free of extremely sharp edges does not cause unnecessary stimulation to the living body and is unlikely to absorb the bone. Inversely, if it is desired to enhance the anchoring effect of an implant to a living hard tissue such as bone for achieving firmer fixation, ceramic particles having sharp edges are preferably used.

Also preferably, the ceramic particles have a coefficient of thermal expansion $\alpha_1$ which is 0.5 to 1.5 times the coefficient of thermal expansion $\alpha_1$ of the metal substrate. This prevents breakage and falling off of ceramic particles during embedding process.

It is believed that ceramic particles are firmly joined to the metal substrate by mechanical engagement while a solid phase bond is created at the interface.

The ceramic particles used herein may be prepared by finely dividing a sintered body, granulating by a pyrolytic spraying, tumbling granulation or fluidized bed process followed by sintering, forming particles by a liquid phase synthesis process using a solution followed by sintering, spray roasting/sintering, or tumbling layer or fluidized bed sintering.

[Ceramic coating]

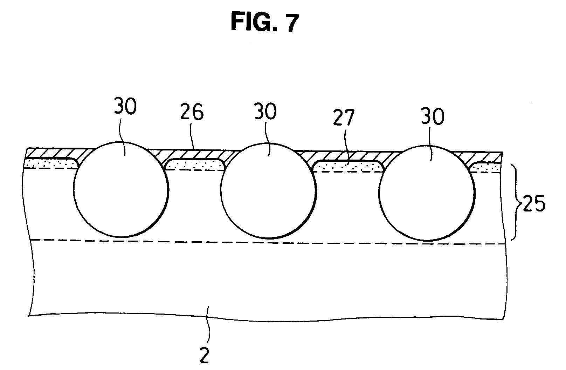

If the metal substrate is not fully covered simply by embedding ceramic particles therein, or if it is desired to completely cover the metal substrate surface with ceramics, then a coating layer 26 of ceramic material can be formed on the embedment layer 25 of ceramic particles as shown in FIG. 7.

The ceramic coating layer can be formed by well-known techniques, for example, a coating technique of mixing ceramic powder with a binder to form a paste and applying and baking the paste, a thermal spraying technique, and vapor phase film deposition techniques including evaporation and sputtering.

The ceramic coating layer can have a strong bond strength which has never been achieved in the prior art because the ceramic coating layer overlies the embedment layer of ceramic particles firmly joined to the metal substrate and forms a ceramic-to-ceramic bond therewith.

The material of which the ceramic coating layer is formed is preferably a ceramic material of substantially the same type as the ceramic particles (a ceramic material based on the same main component), more preferably a ceramic material of the same composition as the ceramic particles in order to bond and integrate the coating layer with the ceramic particles for providing increased bond strength. The same main component means that two ceramic materials contain at least 30% by weight, especially at least 20% by weight, even at least 10% by weight of common components in a weight ratio of from about 1/3 to 3/1, especially from about 1/2 to 2/1 between the two.

The ceramic coating layer overlying the metal substrate generally has a total thickness of 1 to 1,000 $\mu$m, preferably 5 to 500 $\mu$m combined with the embedment layer. A too thin coating layer would be insufficient for the growth of neoblastic bone whereas a too thick coating layer would lead to a lowering of peel strength. This thickness is measured as the thickness between an envelope tangent to the raised portions of the metal substrate surface layer and an envelope tangent to the surface of the coating layer. Preferably in this embodiment, the coating layer is adjusted in thickness such that the surface irregularities defined by the embedment layer of ceramic particles are left behind. Namely, the implant of the invention preferably possesses the rough or irregular surface defined by the embedment layer of ceramic particles, and the ceramic coating layer, when formed, should preferably reflect this surface nature. When the implant is implanted in a living hard tissue, the irregular implant surface is effective for anchoring the implant to the living hard tissue accomplishing firm initial fixation. The irregular configuration presented by bio-affinity ceramic material promotes generation of neoblastic bone which will penetrate among the irregularities to interdigitate therewith, eventually accomplishing a firm fit. The implant on the outer surface preferably has an average surface roughness Ra (JIS B-0601) of 1 to 2,000 $\mu$m, more preferably 5 to 100 $\mu$m.

Further, bio-affinity is enhanced for promoting formation of neoblastic bone by providing the ceramic coating layer with pores to form a porous layer. Then osteoblasts and nutrient vessels will penetrate into pores in the implant surface to promote growth of neoblastic bone, leading to quicker curing.

The pores in the ceramic coating layer preferably have a mean pore diameter of 5 to 100 μm, more preferably 10 to 80 μm. The layer preferably has a porosity of 10 to 70%, more preferably 20 to 60%. A too small average pore diameter impedes entry of cells into pores, failing in quick curing. A too large pore diameter would lower strength and leave a too large gap around cells so that the pores become less effective. A too low porosity would be less effective for improving bio-affinity whereas a too high porosity would lower strength. It is to be noted that the pore diameter and porosity are calculated from an observation under an optical microscope or scanning electron microscope.

The ceramic coating layer can be provided with pores by various prior art well-known methods. For example, ceramic material powder is mixed with a pyrolyzable substance such as cellulose particles and reins particles corresponding to a desired particle diameter and porosity and further with a solvent and resin binder to form a paste, which is coated and baked to an implant. Baking of the ceramic paste causes the pyrolyzable substance particles in the paste to decompose and disappear, leaving pores corresponding to the particles.

The thus formed pores may have various structures depending on the type of pyrolyzable substance mixed. In the case of resin particles, for example, there are left relatively large pores conforming to the particle shape and narrow passage pores created by the escaping gases resulting from decomposition of the resin. In the case of crystalline cellulose or the like, interconnecting irregular pore paths are formed. The pyrolyzable substance particles used herein generally have a mean particle size of 5 to 100 μm, preferably 10 to 80 μm and are mixed in an amount of 10 to 70% by weight, preferably 30 to 60% by weight based on the weight of the ceramic paste.

The method using pyrolyzable substance is rather difficult to form pores of uniform size in uniform distribution. It is thus preferred to control the particle size of glass powder and the heating rate during firing when a crystallized glass method is utilized as will be described later.

Often, the ceramic coating layer baked to the metal substrate preferably has a mean grain size of 0.001 to 100 μm. For materials having a firing temperature of 1000°C or higher, a mean grain size of 0.01 to 50 μm, especially 0.1 to 20 μm is preferred. For lower-temperature firing materials, a mean grain size of up to 1 μm, especially up to 0.1 μm is preferred, the same size being applicable to the coating layer resulting from the crystallized glass method. A too small grain size is difficult to achieve whereas a too large grain size would lower strength. It is to be noted that the grain size is determined by measuring the area of crystal grains by means of a scanning electron microscope (SEM) and calculating the average diameter on the assumption that the grains are circular.

[Low-temperature firable ceramic material]

According to the present invention, when a ceramic coating layer is laminated on an implant having ceramic particles buried therein by a baking process or the like, the temperature of firing the ceramic material should preferably be lower than the melting point of the substrate material.

The melting point of typical metal materials is 1,668°C for metallic titanium, 1,650°C for Ti-6Al-4V alloy, 1,400°C for stainless steel, and 1,300°C for nickel alloys. Therefore, the ceramic firing temperature is preferably up to 1,200°C, more preferably up to 1,000°C.

Low-temperature firing becomes possible by controlling the composition. Alternative useful methods are to increase the activity of ceramics by finely dividing raw material powder, and to mix low-melting glass frit with ceramic powder for lowering the firing temperature. These methods may be used separately, but preferably in combination.

The activity of ceramics can be increased by several methods, for example, by finely dividing raw material powder, and by treating ceramic raw material powder on the surface with acid for activation. The ceramic raw material powder generally has a BET converted particle size value of at least 0.1 m²/g, and it is preferably comminuted to at least 5 m²/g, especially 10 to 200 m²/g when low-temperature firing is necessary. Powder having a too large particle size or a too low BET value is less susceptible to low-temperature firing. Inversely, powder having a too small particle size or a too high BET value is difficult to manufacture. The ceramic material powder should be not only fine, but also uniform in order to provide increased activity.

Material powder as mentioned above may be treated with an acid such as hydrochloric acid prior to firing for increasing its surface activity.

Alternatively, ceramic powder is mixed with low-melting glass frit to form a matrix for lowering the firing temperature. In this method, ceramic powder is mixed with low-melting glass frit with the aid of a solvent such as water to form a paste, which is applied and fired to a substrate for firm bond. Although the addition of glass is effective for lowering the firing temperature, it tends to lower bio-activity. Therefore, the former methods of increasing the activity of ceramics are preferred for bio-activity.

The firing temperature in the last method is above the softening temperature of glass, usually 400 to

EP 0 607 017 A1

1,000°C. Examples of the glass include silica, borate, silicate, borosilicate and phosphate series, with the borosilicate glass being preferred because of appropriate treating temperature. The amount of glass blended is generally 5 to 80% by weight, preferably 15 to 60% by weight based on the total weight of the coating material. Adhesion would lower with a blending amount below the range whereas bio-activity would lower with a blending amount beyond the range.

[Coating method]

The ceramic coating layer may be laminated onto the metal substrate, for example, by baking, thermal spraying or vapor phase film deposition techniques such as sputtering.

The baking method is by mixing the above-mentioned ceramic material powder with a binder component such as organic resins and a solvent component such as alcohols to form a paste, applying the paste to a metal substrate and firing it for baking. This creates a firm bond. The firing temperature is from 500°C to the melting point of the metal substrate, preferably from 800 to 1,550°C, more preferably up to 1,400°C, and up to 1,200°C especially for metal substrates having a low softening point.

The thermal spraying method is by melting ceramic material particles with a gas or plasma and causing the particles in atomized state to deposit on the substrate.

It is to be noted that when conventional HAP materials are deposited by thermal spraying, there arises a problem that the materials are likely to convert into TCP upon heating at high temperatures. Advantageously, the bio-active material series especially preferred in the present invention are unsusceptible to such conversion.

Any intermediate layer may be formed between the metal substrate and the ceramic coating layer. For example, the metal substrate on the surface is oxidized to form a metal oxide film which will form a strong chemical bond with the ceramic coating layer, resulting in increased peel strength.

Insofar as strength is not lost, the ceramic layer can be partially or entirely a porous layer having independent and interconnected pores. It is also possible to form a porous layer on a previously baked dense ceramic layer. This embodiment is effective for promoting retention of osteoblasts and passage of osteoblasts and blood, thereby promoting formation and integration of neoblastic bone.

[Formation of ceramic coating layer by crystallized glass method]

In order to prevent damage to the metal substrate by virtue of low-temperature firing, enhance the adhesion of the ceramic coating layer to the metal substrate, and provide satisfactory bio-affinity, it is preferred to form a coating layer of a non-calcium phosphate system ceramic material based on diopside by a crystallized glass method.

The crystallized glass method involves once melting ceramic powder, cooling the melt for vitrification, comminuting into glass powder, and baking the glass powder to the metal substrate surface, thereby crystallizing at least a portion of vitreous matter to form a ceramic coating layer. Since the baking temperature may be as low as about 800°C, the metal substrate of titanium or the like experiences least oxidation and thus avoids a lowering of its mechanical strength. At the surface of the metal substrate, oxygen and silicon diffuse from the ceramic coating layer to the metal substrate to form a diffusion stratum which contributes to the increased bond strength of the ceramic coating layer. The resulting ceramic coating layer has high bio-affinity. All these ensure that a composite biotic implant has both bio-affinity and mechanical strength.

Where a non-phosphate system ceramic coating layer is formed by a conventional baking method instead of the crystallized glass method, the baking temperature must be increased to about 1,200°C at which the metal substrate is deteriorated and reduced in mechanical strength. In addition, an oxide stratum forms on the metal substrate surface, leaving a risk that the ceramic coating layer can be peeled together with the oxide stratum. Even when a non-phosphate system ceramic coating layer is formed by the crystallized glass method, the crystallization temperature becomes too high if a composition composed mainly of wollastonite forms upon crystallization. For example, JP-A 36107/1992 discloses a crystallized glass composed mainly of $SiO_2$, CaO and MgO and having wollastonite ($CaO \cdot SiO_2$) crystals and diopside ($CaO \cdot MgO \cdot 2SiO_2$) crystals dispersed in glass. One exemplary sample is described therein as consisting of 60% wollastonite and 40% diopside. Although this publication contains no description that a metal substrate is coated with this crystallized glass, we suppose that if coated, crystallized glass containing such a large amount of wollastonite crystals requires heating at 1050°C as taught in the publication. If heat treatment is done at low temperatures of about 800°C at which the metal substrate is not adversely affected, crystallization takes place short and more vitreous matter remains, resulting in a ceramic coating layer having low mechanical strength.

Now description is made how to form a ceramic coating layer by the crystallized glass method.

[Glass powder preparing step]

Glass powder is obtained by melting ceramic material and cooling the melt, followed by comminution.

The melting temperature of ceramic material may be suitably selected depending on the ceramic composition and is generally above 1,400°C. The ceramic material need not previously have a crystalline structure of diopside or the like and may be a mixture of oxides of various components of an intended composition or substances capable of forming such oxides upon melting, for example, carbonates, bicarbonates, and hydroxides. In the latter case, raw materials react with each other upon heating to form a composite oxide. Melting in air is generally acceptable.

The melt of ceramic material is cooled by any desired method which ensures formation of amorphous glass after cooling. For example, the melt is cooled by introducing into water, by contacting with a cooling medium such as a metal chill, or by allowing to cool down in air.

The amorphous glass resulting from cooling is comminuted into glass powder. The size of glass particles is not critical and may be suitably selected depending on the thickness of the ceramic coating layer and the size of pores therein. Preferably the glass particles have a mean particle size of about 1 to 50 μm. Especially for desirably distributing pores of a suitable size in the ceramic coating layer, the glass particles should preferably have a mean particle size of about 2 to 30 μm.

[Baking step]

In the baking step, the glass powder resulting from the glass powder preparing step is mixed with a binder component such as organic resins and a solvent component such as alcohols to form a paste. The paste is coated to the metal substrate and baked to crystallize the glass, forming a ceramic coating layer.

The firing conditions may be suitably selected as long as the glass powder can be crystallized, the metal substrate may not be deteriorated, and an oxide stratum liable to peel is not formed on the metal substrate surface. Preferably the firing temperature is 700 to 900°C, especially 750 to 850°C. At firing temperatures below this range, the glass powder would not be fully crystallized. Beyond the range, a peelable oxide stratum is likely to form on the metal substrate surface and the metal substrate can be deteriorated and reduced in mechanical strength. The holding time is preferably 1/2 to 2 hours, especially 1/2 to 1 hour. A shorter firing time would induce insufficient crystallization of glass powder whereas a longer firing time allows a strippable oxide stratum to form at the metal substrate surface.

For desirably distributing pores of a suitable size in the ceramic coating layer, the heating rate is preferably 2 to 30°C/min.

The atmosphere during firing is generally air or an atmosphere having a similar oxygen partial pressure. If firing is done in a non-oxidizing atmosphere such as vacuum, formation of a diffusion stratum to be described later is insufficient so that the bond strength of the ceramic coating layer may become insufficient. If desired, an atmosphere having a controlled oxygen partial pressure may be used. For example, if an atmosphere having a lower oxygen partial pressure than air is used, the firing temperature can be increased beyond the above-mentioned range without suppressing formation of a diffusion stratum. However, the firing temperature should preferably be below 1,000°C since the metal substrate experiences a substantial loss of strength beyond 1,000°C.

[Diffusion stratum]

With the crystallized glass method used, a diffusion stratum 27 containing oxygen and silicon forms under the surface of the metal substrate after firing (see FIG. 7). This diffusion stratum is effective for increasing the bond strength between the ceramic coating layer and the metal substrate.

While the concentration of oxygen and silicon in the metal substrate decreases from the surface to the center of the metal substrate, the diffusion stratum can be distinguished from the peelable oxide stratum mentioned above by examining the element intensity distribution of oxygen. The element intensity corresponds to the amount of element diffused and can be determined from the count of electron probe microanalysis (EPMA). When only the diffusion stratum is formed, the element intensity of oxygen drastically decreases in substantial proportion to the increasing distance from the substrate surface. On the other hand, when an oxide stratum where an oxide such as $TiO_2$ exists is formed, the element intensity of oxygen is substantially constant from the substrate surface to several tens of microns, which region corresponds to a peelable oxide stratum. The element intensity of oxygen then drastically decreases when one further goes from the oxide stratum toward the substrate center. In this case, an oxide stratum exists under the substrate surface and a diffusion stratum exits inside the oxide stratum.

Provided that the diffusion length of oxygen is the distance from the metal substrate surface to the position where no oxygen is detected by EPMA, a diffusion length of about 2 to 20 μm, especially about 3 to 10 μm is preferred. With a diffusion length below the range, the diffusion stratum is ineffective in increasing the bond strength of the ceramic coating layer. Beyond the range, the oxide stratum can be formed.

The presence of an oxygen-containing region can also be detected from a secondary electron image of a substrate cross section.

[Ceramic material]

Various materials may be used as the ceramic particles to be embedded in a surface layer of a metal substrate and the ceramic coating layer. Exemplary are bio-active ceramic materials, for example, calcium phosphate series such as hydroxyapatite (HAP), tricalcium phosphate (TCP) and bio-glass; monocrystalline and polycrystalline alumina series; zirconia series; and non-calcium phosphate series such as diopside.

The implants intended for use as living body replacements or complements favor ceramic materials having bio-activity in a sense that newly grown neoblastic bone can directly bond with the ceramic material. Known as bio-active ceramic materials are calcium phosphate system ceramic materials to which bone is accessible, typically HAP, TCP and calcium fluoride (FAP).

However, more preferred are bio-active non-calcium phosphate system ceramic materials such as diopside which possess bio-activity and high strength and when indwelled in living tissues, allow bone to grow on a surface so that bonds are created from both the material and bone sides. These materials are sintered ceramic materials having a composition comprising at least one of alkaline earth metal oxides and alkali metal oxides and $SiO_2$ and at the same time, non-calcium phosphate system sintered ceramic materials which are substantially free of phosphorus as a base component. Irrespective of the non-calcium phosphate system, these materials are biologically active and characterized in that upon contact with an aqueous solution containing phosphorus (e.g., spurious and true body fluids), they form calcium phosphate system compounds, typically hydroxyapatite (HAP) on their surfaces of contact.

Ceramic materials having bio-compatibility or bio-affinity are acceptable when medical instruments or implements to be indwelled in living bodies are contemplated. The bio-compatibility or bio-affinity means that bone can grow in contact with the ceramic material without leaving gaps. Since bio-activity is a special example of bio-compatibility, the bio-compatibility is used in this specification as encompassing bio-activity.

Almost all ceramic materials are useful as the bio-compatible ceramic materials, including oxide ceramic materials such as alumina, zirconia, silica, calcia, magnesia, and titania series materials, carbide series materials and nitride series materials. For medical instruments or implements to be indwelled in living bodies, these materials are applicable to both the ceramic particles to be embedded and the ceramic coating layer. For living hard tissue replacements or complements, these materials are applicable to the ceramic particles embedded which are covered with the ceramic coating layer. However, since the coating layer should preferably have bio-activity and the particles should preferably be of the same type of material, it is preferred that both the particles to be embedded and the coating layer are of a bio-active ceramic material which will be described below.

[Bio-active composition]

Typical of the bio-active ceramic material used herein is a composition comprising at least one member of alkaline earth metal oxides and alkali metal oxides and $SiO_2$ in a weight ratio of from 1:4 to 6:1, preferably from 1:3 to 2:1. Outside the range, bio-affinity or strength lowers.

This composition can be reduced in coefficient of thermal expansion by increasing the content of $SiO_2$ relative to the content of alkaline earth and alkali metal oxides. This will advantageously provide a matching in coefficient of thermal expansion between the ceramic material and the metal substrate as previously described (with the ratio therebetween ranging from 1/2 to 3/2). If the metal substrate is noticeably different from the ceramic material in coefficient of thermal expansion, the ceramic portion can be broken or damaged during metal plastic working or baking of the ceramic coating layer. Where a ceramic material is laminated on the metal substrate, its coefficient of thermal expansion $\alpha_1$ can be adjusted to the range of from $6.65 \times 10^{-6}$ to $12.35 \times 10^{-6}/°C$ by controlling the content of $SiO_2$ in the range of 30 to 75% by weight, preferably 35 to 70% by weight of the entire ceramic composition.

In these cases, the alkaline earth metal oxide is one or two members selected from CaO, MgO, SrO, BaO, etc., with CaO and MaO being preferred.

[CaO essential composition]

Ceramic materials containing CaO capable of precipitating HAP component, among other alkaline earth metal oxides, as an essential component are preferred for bio-activity, strength and ease of manufacture. Useful are ceramic compositions containing 20 to 90% by weight, especially 30 to 70% by weight of CaO.

It is also possible to use CaO as an essential component and partially another alkaline earth metal oxide such as MgO, SrO and BaO. Especially, inclusion of MgO is preferred since it contributes to lower-temperature firing capability and the adjustment of coefficient of thermal expansion. In the composition represented by $xCaO \cdot yMgO \cdot 2SiO_2$, increasing x will increase the coefficient of thermal expansion and increasing y will decrease the coefficient of thermal expansion. For example, the compositions of $CaO \cdot 2SiO_2$, $1/2CaO \cdot 1/2MgO \cdot 2SiO_2$, and $MgO \cdot 2SiO_2$ have an $\alpha_1$ of $10.0 \times 10^{-6}$, $9.5 \times 10^{-6}$, and $7.5 \times 10^{-6}$, respectively. The coefficient of thermal expansion can be adjusted in this way. The weight ratio of CaO to MgO preferably ranges from 1:10 to 100:0, especially from 1:10 to 10:1.

The content of MgO is preferably in the range of 0.1 to 60% by weight of the ceramic composition. For matching of coefficient of thermal expansion with such metals as Ti and lower-temperature firing capability, the content of MgO should be in the range of from 0.1 to 35% by weight. Materials mainly composed of CaO preferably have a composition comprising 10 to 88% by weight of CaO, 2 to 35% by weight of MgO, and 10 to 80% by weight of $SiO_2$, more preferably 18 to 47% by weight of CaO, 10 to 25% by weight of MgO, and 37 to 68% by weight of $SiO_2$.

[CaO-free composition]

At the beginning, we believed that the presence of CaO containing a HAP component is indispensable to form HAP on ceramics. Quite unexpectedly, our continuing study revealed that a compositional system free of CaO also has an ability to form HAP. This compositional system was found to be more bio-active than the conventional calcium phosphate system ceramics.

More particularly, it is possible to use instead of CaO, at least one metal oxide selected from other alkaline earth metal oxides, e.g., MgO, SrO and BaO and/or alkali metal oxides. Compositions substantially free of CaO are available in some cases. Where other alkaline earth metal oxides such as MgO, SrO and BaO are used, their content may range from 0.1 to 90% by weight of the ceramic composition and preferably their total content ranges from 20 to 90% by weight, especially from 30 to 70% by weight of the ceramic composition.

Alkali metal oxides may be used instead of the alkaline earth metal oxides or as a partial substitute therefor. In this case, one or more members are selected from $Na_2O$, $K_2O$ and $Li_2O$ and they are preferably used as an additive component to MgO and sometimes CaO.

The content of alkali metal oxides may range from 0.1 to 90% by weight of the ceramic composition and preferably their total content ranges from 0.1 to 70% by weight, especially up to 50% by weight of the ceramic composition from the standpoints of strength, bio-activity and matching of coefficient of thermal expansion.

[Exemplary composition field]

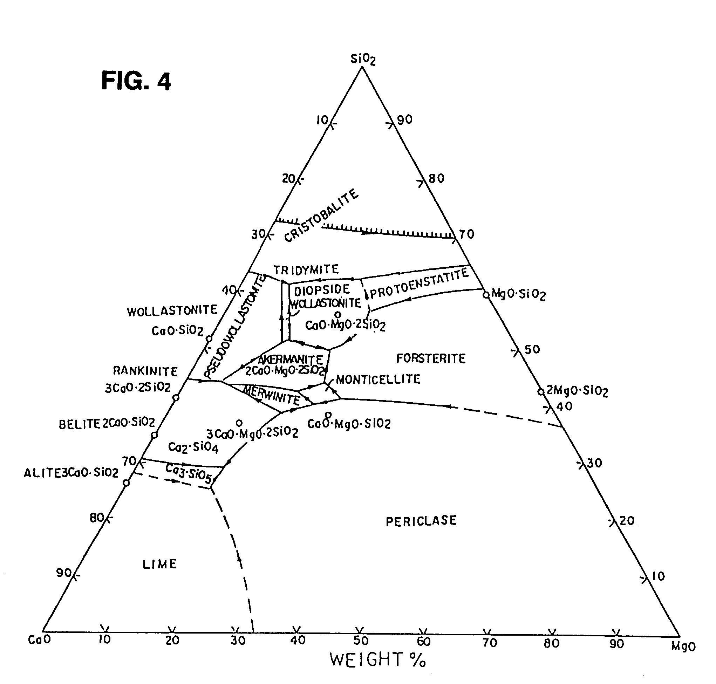

The ceramic materials used herein, if they are of material series containing alkaline earth metal oxides, are ceramic materials belonging to the fields of diopside: $(Ca,Mg)O-MgO-2SiO_2$, especially $2SiO_2-CaO-MgO$, wollastonite: $\beta-(Ca,Mg)O-SiO_2$, especially $CaO-SiO_2$, alite: $3CaO-SiO_2$, belite: $2CaO-SiO_2$, akermanite: $2CaO-MgO-2SiO_2$, monticellite: $CaO-MgO-SiO_2$, forsterite: $2(Mg,Ca)O-SiO_2$, proteoenstatite: $(Mg,Ca)O-SiO_2$, tridymite: $SiO_2$ and so on. These fields are depicted in the ternary phase diagram of FIG. 4.

Preferred material series containing CaO as an essential component are those belonging to the diopside, wollastonite, alite, belite, akermanite, and monticellite fields, and among others, ceramic materials predominantly comprising those belonging to the diopside field and capable of firing at relatively low temperatures and those belonging to the wollastonite field are especially preferred with the additional benefit of high strength. In the case of the CaO-free material series, those belonging to the forsterite field are preferred. In addition to the ceramic materials belonging to the selected compositional fields, a mixture thereof with another compound as mentioned above is also useful.

Among material series containing alkali metal oxides are $SiO_2-K_2O$, $SiO_2-LiO-MgO$, $SiO_2-Li_2O-TiO_2$, $SiO_2-TiO_2-CaO$, $SiO_2-Na_2O$ and similar compositional series. Those which can be sintered at low temperatures are $SiO_2-K_2O$ and $SiO_2-Na_2O$ series.

In addition to the aforementioned components, the ceramic materials which can be used herein may have blended therein an optional component such as $TiO_2$, ZnO, $B_2O_3$, FeO, and $ZrO_2$, if necessary, in an amount not impairing the desired physical properties. The bond strength of the ceramic material to the metal substrate can be increased by introducing an oxide of a metal substrate component such as $TiO_2$ into the ceramic material. It will be understood that inclusion of $Al_2O_3$ is less desirable because of its detrimental influence on bio-activity.

[Ceramic coating layer composition suitable for the crystallized glass method]

The most preferred ceramic coating layer used herein has a non-calcium phosphate system composition containing silicon oxide, calcium oxide, and magnesium oxide, contains diopside crystals and is formed by the crystallized glass method.

In implants as typified by living body replacements and complements, it is desired that neoblastic bone directly bond with the implant surface material. That is, it is desired that the implant surface is bio-active. The ceramic coating layer having a higher diopside content has bio-activity and strength and allows new bone to grow thereon when the implant is indwelled in the living tissue whereby bonds are created from both the material and bone sides. Upon contact with an aqueous solution containing phosphorus (e.g., spurious and true body fluids), this material forms calcium phosphate system compounds, typically hydroxyapatite (HAP) on its surface of contact.

The diopside in the ceramic coating layer has a composition: $(Ca,Mg)O\text{-}MgO\text{-}2SiO_2$, especially $2SiO_2\text{-}CaO\text{-}MgO$. In the present invention, the ceramic coating layer favors that diopside occupies at least 50%, more preferably at least 70%, most preferably 100% by volume of the entire crystalline matter. A ceramic coating layer with a less proportion of diopside has low mechanical strength.

Useful crystals which can appear other than diopside include wollastonite: $\beta\text{-}(Ca,Mg)O\text{-}SiO_2$, especially $CaO\text{-}SiO_2$, alite: $3CaO\text{-}SiO_2$, belite: $2CaO\text{-}SiO_2$, akermanite: $2CaO\text{-}MgO\text{-}2SiO_2$, monticellite: $CaO\text{-}MgO\text{-}SiO_2$, forsterite: $2(Mg,Ca)O\text{-}SiO_2$, proteoenstatite: $(Mg,Ca)O\text{-}SiO_2$, and tridymite: $SiO_2$ as depicted in the ternary phase diagram of FIG. 4.

The ceramic coating layer is generally comprised of a diopside base crystalline matter dispersed in a vitreous matrix. The proportion of the crystalline matter in the ceramic coating layer is preferably at least 30%, more preferably at least 50%, most preferably at least 90% by volume. A ceramic coating layer with a less proportion of the crystalline matter would have insufficient mechanical strength.

It is noted that the proportion of crystalline matter in the ceramic coating layer and the proportion of diopside in the crystalline matter can be determined by a peak separation technique using an X-ray diffraction chart. In an X-ray diffraction chart of crystallized glass having crystals dispersed in a vitreous matrix, there appear a halo indicative of the presence of vitreous matter and inherent peaks corresponding to a particular crystalline matter. The peak separation technique determines an integral intensity as a sum of only peak areas and an overall integral intensity as a sum of peak and halo areas and divides the former by the latter to calculate a degree of crystallinity.

The proportion of diopside in the crystalline matter is calculated by determining an integral intensity of peaks attributable to diopside and dividing this by the overall integral intensity.

In the ceramic coating layer, any desired component such as $TiO_2$, $ZnO$, $B_2O_3$, $FeO$ and $ZrO_2$ may be additionally blended in an amount not to detract from desired physical properties, typically in an amount of less than 5% by weight. Especially by incorporating an oxide of metal substrate material such as $TiO_2$ into the ceramic material, the bond strength to the metal substrate can be increased. Containment of $Al_2O_3$ is not recommended since it tends to lower bio-activity.

[Synthesis of ceramic material]

The ceramic material powder used herein may be synthesized by dry and wet synthesis methods or the like as mentioned above. Preferred for producing fine uniform powder are a pyrolytic spraying method, liquid phase synthetic method such as co-precipitation and precipitation, alkoxide method, and sol-gel method.

More particularly, the pyrolytic spraying method is by atomizing an aqueous solution containing ceramic component ions adjusted to a desired composition with the aid of gas or a ultrasonic vibrator, and heating the droplets for synthesizing spherical, hollow, fine particles. The hollow particles may be ground for further increasing the BET value.

The co-precipitation method is by evenly mixing ceramic component ions in an aqueous solution state and allowing mixed components to chemically precipitate as a solid phase concurrently by virtue of differential solubility. There are obtained fine particles of high purity and at least 60 $m^2/g$.

The alkoxide method is by mixing a Ca alkoxide, a Si alkoxide and the like to form an alkoxide solution containing respective ceramic components, and subjecting the solution to hydrolysis reaction for synthesizing fine particles of high purity and high BET value.

The sol-gel method is by mixing selected components in aqueous solution form to form a sol, dewatering the sol into a gel, and calcining the gel into oxides.

EXAMPLE

Examples of the present invention are given below by way of illustration and not by way of limitation.

A number of artificial dental root samples were fabricated as reported in Table 1.

The superplastic metal material used as the substrate was pure metallic titanium having a ductility of at least 200% at the temperature (1,000°C) corresponding to 70% of its melting point. It was shaped as shown in FIG. 2. The artificial dental root substrate was dimensioned to have a length of 13.5 mm, a root diameter (stem diameter) of 2.7 mm, and a cervical diameter (head diameter) of 3.7 mm and roughened on the surface by blasting to Ra = 35 $\mu$m.

Ceramic particles were embedded in the outer surface of the substrate to form an embedment layer. The ceramic particles to be embedded were obtained by mixing $CaCO_3$, $SiO_2$ and MgO in a conventional manner, calcining the mixture at 1,000°C, milling, spray drying for granulation, and then firing at 1,280°C. The fired product was classified through screens to collect a fraction of dense diopside ($CaO \cdot 2SiO_2 \cdot MgO$) particles having a mean particle size and sphericity as reported in Table 1. The diopside particles had a compression strength of 200 MPa and a coefficient of thermal expansion of $10 \times 10^{-6}$/°C, which is 1.0 times the coefficient of thermal expansion of the titanium metal substrate equal to $9.7 \times 10^{-6}$/°C.

As shown in FIG. 2, high vacuum grease was applied to the surface of metal substrate 2 and ceramic particles 30 of the above-mentioned diopside were adhered to the entire side and bottom surfaces of substrate 2. Hot pressing was uniaxially effected in a vacuum of $8 \times 10^{-5}$ Torr by means of a hot press assembly for radially extending the metal substrate for providing plastic working. The press assembly used is shown in FIGS. 2 and 3a. The split mold 4 was of alumina and the cylindrical sleeve 5 was of silicon nitride. The substrate 2 was entirely fitted in the mold cavity. Pressing was conducted by first heating the substrate 2 fitted in the mold from room temperature to 950°C over one hour, then applying a pressure of 100 MPa for 30 minutes while maintaining the temperature, and then allowing to cool down to room temperature over one hour. By this hot pressing, ceramic particles were forced into the substrate surface to form an embedment layer.

For comparison purpose, samples (sample Nos. 6 and 7) were fabricated using a conventional hot press assembly of the arrangement shown in FIG. 3b. The metal substrate 2 protruded above the split mold 4 when the punch 61 forced the substrate 2 toward the support 65. By hot pressing under otherwise identical conditions, ceramic particles were forced into the substrate surface to form an embedment layer.

Table 1 also reports the spacing (maximum spacing) between the ceramic particle surface and the mold cavity surface when the dental root substrate was inserted into the mold cavity.

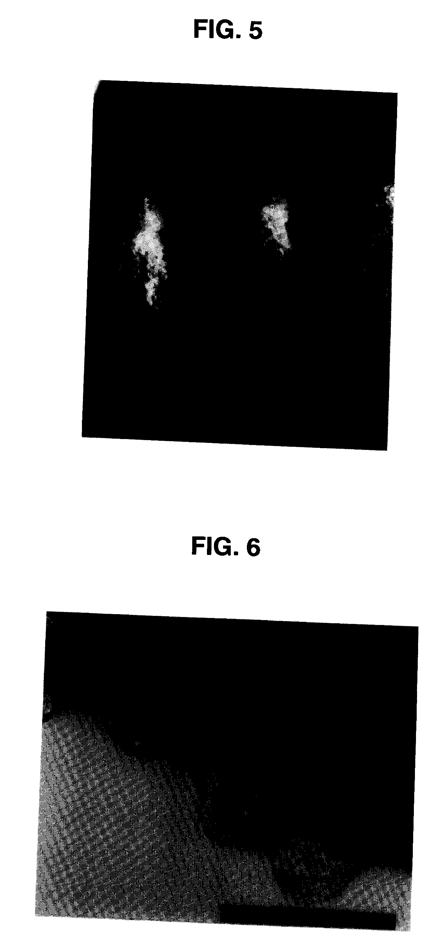

FIGS. 5 and 6 are SEM photomicrographs in cross section of the embedment layers of sample Nos. 1 and 7 among the artificial dental root substrates having ceramic particles embedded.

The artificial dental root substrates having an embedment layer were measured for percent embedment depth. They were also measured for dimensions A and B and angle $\theta$ as shown in FIGS. 1a and 1b. Measurements meeting the requirement of the invention are labeled "O" and outside measurements are labeled "X". The percent of those recesses meeting A < B or $\theta$ < 90° (for at least one end) is reported in parentheses.

Next, a ceramic coating layer was formed over the embedment layer. First, $SiO_2$, $CaCO_3$ and MgO were milled in a ball mill for one hour and melted in a platinum alloy crucible by heating at 1,400°C for one hour. The melt was quenched in water, obtaining amorphous glass frit. The glass frit was milled in a vibratory mill and then in a sand mill for 2 hours, obtaining glass powder having a mean particle size of 5 $\mu$m. A paste was prepared by mixing the glass powder with 3 wt% metrose aqueous solution in a weight ratio of 1:1. The paste was applied to the surface of the substrates, heated at a rate of 10°C/min., and fired in air at 800°C for one hour to form a ceramic coating layer of about 5 $\mu$m thick, obtaining artificial dental root samples. The thickness of the ceramic coating layer was measured from a photomicrograph of a cross section. The ceramic coating layer consisted of 55% of $SiO_2$, 26% of CaO and 19% of MgO, by weight, had a crystallinity of 95% by volume. The crystallized grains were of diopside. The crystallinity was determined by the peak separation technique previously mentioned. The diffusion length of oxygen was 10 $\mu$m from the substrate surface and no oxide stratum was found. The diffusion length of oxygen and the presence of an oxide stratum were detected using EPMA as mentioned above.

The artificial dental root samples were measured for peel strength and durability in the living body. The peel strength was determined by inserting a sample through an epoxy resin ring with its opposite end surfaces exposed, and pressing the sample between upper and lower punches. While the pressing force was gradually increased, the pressure force at which release of ceramic particles or peeling of the ceramic coating layer was detected is the peel strength.

The durability in the living body was examined by implanting artificial dental root samples in openings perforated in the jaw bones of rabbits. The jaw bones were removed 6 months later and prepared into specimens which were observed in section to examine release of ceramic particles and peeling of the ceramic coating layer. Durability is evaluated in accordance with the following criterion.

O: no release of ceramic particles and no peeling of ceramic coating layer after 6 months

X: release of ceramic particles and/or peeling of the ceramic coating layer observed after 6 months

The results are shown in Table 1.

Table 1

| Sample No. | Ceramic particles Mean particle size (μm) | Spheri-city | Embedment depth (%) | Press assembly | Spacing (mm) | A<B (%) | Angle θ (%) | Peel strength (MPa) | Dura-bility |
|---|---|---|---|---|---|---|---|---|---|
| 1 | 125-150 | 1.07 | 100 | FIG. 3a | 0.01 | O (20) | O (25) | 750 | O |
| 2 | 125-150 | 1.07 | 65 | FIG. 3a | 0.02 | O (10) | O (15) | 750 | O |
| 3 | 100-125 | 1.07 | 70 | FIG. 3a | 0.1 | O (15) | O (20) | 780 | O |
| 4 | 125-150 | 1.13 | 60 | FIG. 3a | 0.02 | O ( 5) | O ( 5) | 730 | O |
| 5* | 125-150 | 1.48 | 60 | FIG. 3a | 0.02 | X ( 1) | X ( 2) | 600 | X |
| 6* | 125-150 | 1.07 | 60 | FIG. 3b | 0.05 | X ( 0) | X ( 0) | 550 | X |
| 7* | 125-150 | 1.07 | 100 | FIG. 3b | 0.01 | X ( 0) | X ( 0) | 530 | X |

* comparison

The effectiveness of the present invention is evident from the data of Table 1.

Although some preferred embodiments have been described, many modifications and variations may be made thereto in the light of the above teachings. It is therefore to be understood that within the scope of the appended claims, the invention may be practiced otherwise than as specifically described.

## Claims

1. A composite biotic implant comprising a metal substrate having a surface layer where ceramic particles are embedded and received in recesses,

   wherein in a cross section of the embedment layer, at least 5% of the recesses satisfy A < B wherein A is the distance between opposed ends of the outside edge of the recess in which a ceramic particle is received, and B is the maximum diameter of the ceramic particle as measured perpendicular to a normal to the metal substrate surface.

2. A composite biotic implant according to claim 1 wherein said metal substrate is made of a metal exhibiting a ductility of at least 50% at a temperature corresponding to 70% or less of its melting point.

3. A composite biotic implant according to claim 1 which further comprises a ceramic coating layer disposed on the embedment layer.

4. A composite biotic implant according to claim 3 wherein said ceramic coating layer has a non-calcium phosphate system composition containing silicon oxide, calcium oxide, and magnesium oxide wherein diopside crystals occupy at least 50% by volume of the entire crystalline matter, the surface layer of the metal substrate includes a diffusion stratum containing oxygen and silicon.

5. A composite biotic implant comprising a metal substrate having a surface layer where ceramic particles are embedded and received in recesses,

   wherein in a cross section of the embedment layer, at least 5% of the recesses satisfy that at least one of opposed ends of the outside edge of the recess has an included angle of less than 90°.

6. A composite biotic implant according to claim 5 wherein said metal substrate is made of a metal exhibiting a ductility of at least 50% at a temperature corresponding to 70% or less of its melting point.

7. A composite biotic implant according to claim 5 which further comprises a ceramic coating layer disposed on the embedment layer.

8. A composite biotic implant according to claim 7 wherein said ceramic coating layer has a non-calcium phosphate system composition containing silicon oxide, calcium oxide, and magnesium oxide wherein diopside crystals occupy at least 50% by volume of the entire crystalline matter, the surface layer of the metal substrate includes a diffusion stratum containing oxygen and silicon.

9. A composite biotic implant according to any one of claims 1 to 8 which is prepared by a process comprising the steps of using a hot press assembly including a mold defining a cavity and a punch, applying ceramic particles to the surface of a metal substrate, fully inserting the metal substrate into the mold cavity, and forcing the punch against the substrate and into the mold cavity to uniaxially pressurize the metal substrate, thereby embedding the ceramic particles in the metal substrate.

10. A composite biotic implant according to claim 9 wherein when the metal substrate has been fully inserted into the mold cavity, the ceramic particles on the surface are spaced a distance of up to 0.5 mm from the mold cavity surface.

11. A method for preparing a composite biotic implant comprising the steps of using a hot press assembly including a mold defining a cavity and a punch, applying ceramic particles to the surface of a metal substrate, fully inserting the metal substrate into the mold cavity, and forcing the punch against the substrate and into the mold cavity to uniaxially pressurize the metal substrate, thereby embedding the ceramic particles in the metal substrate.

12. A method for preparing a composite biotic implant according to claim 11 wherein when the metal substrate has been fully inserted into the mold cavity, the ceramic particles on the surface are spaced a distance of up to 0.5 mm from the mold cavity surface.

## FIG. 1a

(a)

## FIG. 1b

(b)

# FIG. 2

# FIG. 3a
## INVENTION

67

61

2

4

5

65

# FIG. 3b
## COMPARISON

67

61

2

4

5

65

FIG. 4

## FIG. 5

## FIG. 6

# FIG. 7

European Patent
Office

**EUROPEAN SEARCH REPORT**

Application Number

EP 94 30 0181

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,X | WO-A-92 14422 (TDK CORPORATION) & EP-A-0 525 210 (TDK CORPORATION) * abstract; claims 1,2,7,10,15,18,19,22; figures; table 1 * | 1-9,11 | A61F2/30 |
| X | EP-A-0 006 544 (BATTELLE-INSTITUT) * abstract; figures * | 1-3,5-7, 9,11 | |
| X | WO-A-87 06842 (ERNST LEITZ WETZLAR) * abstract; claims 1-11 * | 1-9,11 | |
| A | EP-A-0 280 592 (COMMISSARIAT A L'ENERGIE ATOMIQUE) | | |

TECHNICAL FIELDS
SEARCHED      (Int.Cl.5)

A61F
A61C

The present search report has been drawn up for all claims

| Place of search | Date of completion of the search | Examiner |
|---|---|---|
| THE HAGUE | 14 April 1994 | Wolf, C |

EPO FORM 1503 03.82 (P04C01)